# EUROPEAN PATENT APPLICATION

(11) **EP 4 603 584 A1**
(43) Date of publication of application: **20.08.2025**
(21) Application number: 24158129.7
(22) Date of filing: 16.02.2024
(51) Int. Cl.: C12N 15/11

(54) **AFFINITY REAGENT, MARKER AND METHOD FOR ANALYSING A BIOLOGICAL SAMPLE**

(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

An affinity reagent (102, 102a, 102b) for analysing a biological sample is provided. The affinity reagent (102, 102a, 102b) comprises a nucleic acid backbone (200, 300, 402). The nucleic acid backbone (200, 300, 402) is configured to specifically bind to a target analyte (106) by its complex structure (400c). Further, the nucleic acid backbone (200, 300, 402) comprises at least one pair of reaction moieties (202, 204, 302, 304, 308, 310) for cross-linking the nucleic acid backbone (200, 300, 402). In further aspects, a marker and a method for analysing a biological sample are provided.

## Description

### Technical field

The invention relates to an affinity reagent for analysing a biological sample. In further aspects, a marker comprising the affinity reagent and a method for analysing a biological sample with the marker are provided.

### Background

Nucleic acid based affinity reagents are superior to antibodies in many regards. For example, aptamer discovery through SELEX is easy and cost-effective. Further, the information about the nucleic acid based affinity reagents can be digitally stored and can be used to produce the respective nucleic acid based affinity reagent by oligonucleotide synthesis with excellent batch-to-batch variability at low cost. They generally exhibit high affinities and are easy to functionalize and multimerize.

A key challenge, however, remains unresolved. Nucleic acid based affinity reagent binding depends on their 3D structure. The stability of nucleic acid based 3D structures is however lower than protein-based 3D structures owing to lack of side-chain interactions. For this reason, a given aptamer may dynamically change between different 3D structures, of which only one or few are active and specifically bind to the target molecule. Similarly, during storage nucleic acid based affinity reagents may partially unfold or misfold and may thus need to be refolded prior to use. This significantly reduces the ease of use and practical applications of nucleic acid based affinity reagents.

### Summary

It is an object to provide an affinity reagent and a marker that are robust and a method for analysing a biological sample with the marker.

The aforementioned object is achieved by the subject-matter of the independent claims. Advantageous embodiments are defined in the dependent claims and the following description.

In a first aspect, an affinity reagent for analysing a biological sample is provided. The affinity reagent comprises a nucleic acid backbone. The nucleic acid backbone is configured to specifically bind to a target analyte by its complex structure. Further, the nucleic acid backbone comprises at least one pair of reaction moieties for cross-linking the nucleic acid backbone. This enables providing a robust and stable affinity reagent.

The nucleic acid backbone may be a deoxyribonucleic acid molecule, for example. In particular, the nucleic acid backbone may be a single, continuous deoxyribonucleic acid molecule. The nucleic acid backbone may have an unfolded, primary structure that is a linear sequence of nucleotides, for example, linked together by phosphodiester bonds.

In a preferred embodiment, I nucleic acid backbone may entirely or partially comprise a xeno nucleic acid backbone, i.e. the backbone may comprise phosphorothioate, boranophosphate, squaramides, or triazole linkages. Such backbones may confer higher stability and resistance against degradation. Likewise, the nucleic acid backbone may comprise L-DNA and/or modified nucleobases, including nucleobases that have side chain-like residues, as is the case for SOMAmers, for example.

The complex structure or conformation of the nucleic acid backbone of the affinity reagent may be a secondary, tertiary or quaternary structure of the nucleic acid backbone, for example. Thus, the nucleic acid backbone of the affinity reagent may preferably have a secondary, tertiary, or quaternary structure. In particular, the affinity reagent is configured to bind specifically to the target analyte, for example of the biological sample, due to the complex structure of the backbone of the affinity reagent. Specifically, individual, discontinuous nucleotides of the nucleic acid backbone bind to the target analyte, rather than continuous nucleotides of a sequence of the nucleic acid hybridising to the target analyte, in particular a complementary sequence of the target analyte. This enables binding of the affinity reagent with high specificity, for example to an amino acid based target analyte. In particular, the backbone of the affinity reagent, e.g. an aptamer, may bind to its target by paratope-epitope interactions and not via hybridization of complementary nucleic sequences. The binding of the affinity reagent to the target analyte may comprise intermolecular forces such as hydrogen bonding, dipole-dipole interactions, ionic interactions, π-stacking, hydrophobic interaction, and van der Waals forces.

The reaction moieties of the pair of reaction moieties may be arranged along the linear sequence of nucleotides of the nucleic acid backbone, for example, with each reaction moiety attached to two nucleotides of the linear sequence. Thus, the reaction moieties are arranged along the nucleic acid backbone sequence not adjacent to each other. However, in the secondary, tertiary and/or quaternary structure of the nucleic acid backbone, the position of the reaction moieties relative to each other may change due to a corresponding change in conformation of the nucleic acid backbone. Preferably, the reaction moieties are at a smaller distance to each other when the nucleic acid backbone has the complex structure compared to when the nucleic acid backbone has the unfolded, linear primary structure.

In particular, the nucleic acid backbone may comprise a cross-link between the at least one pair of reaction moieties. In particular, the cross-link and/or the at least one pair of reaction moieties is for maintaining the complex structure of the nucleic acid backbone. To that end, the reaction moieties and/or the cross-link preferably cross-link positions along the nucleic acid backbone that are not adjacent along the nucleic acid backbone. For example, the reaction moieties of the pair of reaction moieties are arranged along the linear sequence of the nucleic acid backbone at a distance from each other, as discussed above. Thus, an intrastrand cross-link may be formed, in particular, comprising the reaction moieties of the pair of reaction moieties. The intrastrand cross-link may cross-link two separate positions along the linear sequence of a nucleic acid molecule of the nucleic acid backbone, for example.

In a preferred embodiment, the nucleic acid backbone may comprise multiple nucleic acid molecules that bind and fold together to form the complex structure. In this case, the cross-link may be formed between the reaction moieties of the pair of reaction moieties, which are arranged on different ones of the multiple nucleic acid molecules. This may be referred to as an interstrand cross-link.

Preferably, the reaction moieties of the pair of reaction moieties are configured to react with each other. This may result in formation of a covalent bond between the reaction moieties and enables a robust affinity reagent with a stable complex structure. In particular, the reaction moieties are configured to react specifically or directly with each other. This enables forming the covalent bond specifically between the reaction moieties. Thus, the reaction moieties preferably attach to each other covalently, i.e. they react with each other to form a covalent bond, and the reaction do not bind to each other by hybridisation or by intermolecular forces such as hydrogen bonding, dipole-dipole interactions, and van der Waals forces. The reaction of the reaction moieties may in particular be a copper-dependent azide alkyne cycloaddition (CuAAC), a strain-promoted azide alkyne cycloaddition (SpAAC), a Staudinger reaction, Diels-Alder, thiole-ene reaction, or any other suitable reaction. The reaction moieties may be in particular, azide, alkyne, dibenzocyclooctyne (DBCO), thiol, ene, maleimide, N-hydrocysuccinimide, amino.

Preferably, the reaction moieties of the pair of reaction moieties are configured to react under a catalytic condition, in particular only under the catalytic condition. This enables control over the reaction of the reaction moieties of the pair of reaction moieties. For example, the catalytic condition may be the presence of a catalyst. In particular, the catalyst may be light such as UV-light, copper ions, or a molecule.

Preferably, the affinity reagent further comprises a linking element and the reaction moieties of the pair of reaction moieties are configured to react with corresponding reaction moieties of the linking element. This enables a flexible cross-linking of the nucleic acid backbone. For example, the linking element may be chosen depending on the distance between the reaction moieties of the pair of reaction moieties when the nucleic acid backbone has the complex structure. The linking element may comprise a polyethylene glycol (PEG) molecule, which are available in a variety of lengths. Further, the corresponding reaction moieties of the linking element may be click chemistry groups or NHS groups. The PEG molecule may be functionalised accordingly. Each of the reaction moieties of the pair of reaction moieties preferably forms a covalent to one of the corresponding reaction moieties of the linking element.

Preferably, the reaction moieties of the pair of reaction moieties are modified, in particular functionalised, nucleotides. This enables efficient integration of the reaction moieties in the nucleic acid backbone. For example, the reaction moieties may be non-natural or artificial nucleotides, in particular nucleobases, that preferably do not bind by base-pairing to the natural nucleotides adenine, thymine, guanine, and cytosine.

Preferably, the reaction moieties of the pair of reaction moieties comprise click chemistry groups. This enables efficient generation of the cross-link. For example, the reaction moieties may be CuAAC with azide/alkyne groups, or SpAAC with DBCO/azide groups. For example, in case of CuAAC, the catalytic condition may be the presence of a copper catalyst.

Preferably, the reaction moieties of the pair of reaction moieties are photoreactive. This enables efficient generation of the cross-link. For example, the reaction moieties may be photoreactive nucleobases. In a particular example each of the reaction moieties is a thymine nucleotide, which reacts under UV-light to form a thymine dimer.

Preferably, the nucleic acid backbone comprises 10 to 150 nucleotides. This enables a particular compact affinity reagent that is able to efficiently penetrate a biological sample such as a tissue section.

Preferably, the nucleic acid backbone is an aptamer. This enables a compact and robust affinity reagent. Further, this enables efficient generation of the nucleic acid backbone that specifically binds to a particular target analyte, for example by a SELEX method.

Preferably, the reaction moieties of the pair of reaction moieties are separated from each other along the nucleic acid backbone. This enables providing a robust nucleic acid backbone with a stable cross-link. In particular, the reaction moieties are separated from each other along a primary structure or sequence of the nucleic acid backbone. Thus, the reaction moieties are not immediately adjacent to each other or in proximity along the sequence of the nucleic acid backbone.

Preferably, the reaction moieties of the pair of reaction moieties are in proximity when the affinity reagent has its complex structure. This enables providing a robust nucleic acid backbone with a stable cross-link. Thus, the reaction moieties are arranged along the nucleic acid backbone at positions that are in spatial proximity when the nucleic acid backbone is in its complex structure. The spatial proximity of the reaction moieties enables a reaction of the reaction moieties with each other, in particular.

Preferably, the affinity reagent comprises a second pair of reaction moieties, wherein the reaction moieties of the second pair differ from the reaction moieties of the at least one pair of reaction moieties. This enables generating a robust affinity reagent. In particular, the reaction moieties of the second pair differ from the reaction moieties of the at least one pair of reaction moieties in that they do not react with each other. Thus, only the reaction moieties of the second pair react with each other and the reaction moieties of the at least one pair of reaction moieties react with each other.

In a preferred embodiment, the affinity reagent may comprise at least one further pair of reaction moieties, wherein the further pair of reaction moieties is different to the first and second pair of reaction moieties.

In a further aspect, a marker for analysing a biological sample is provided. The marker comprises an affinity reagent as detailed above and a label comprising at least one labelling moiety. The labelling moiety may be attached to the affinity reagent. Preferably, the labelling moiety is optically detectable, for example, a fluorophore. The affinity reagent enables providing a robust marker, that may be coupled with heat-sensitive labelling moieties, for example. In particular, the requirement to refold nucleic acid based affinity reagents after storage by heat may negatively impact the heat-sensitive labelling moieties.

In another aspect, a method for analysing a biological sample is provided. The method comprises the steps: introducing into the biological sample at least one marker, for example as described above, and generating a readout of the biological sample with the marker.

Individual elements of the at least one marker may be introduced individually into the biological sample, for example. Thus, the affinity reagent and the label of the marker may be introduced separately, in order to generate the marker in the biological sample. The readout is preferably an optical readout, for example, generated by means of a microscope.

Preferably, prior to introducing the at least one marker, the affinity reagent of the marker is generated by cross-linking the pair of reaction moieties of the marker. Thus, a cross-linking of the pair of reaction moieties of the affinity reagent is effected. This may include applying the catalytic condition.

Generally, a plurality of markers may be introduced into the biological sample, the markers being specific to respective target analytes, in order to identify a large number of (different or the same) target analytes at the same time. Preferably, a target analyte is identified and/or localised within the biological sample based on the label(s) of the marker(s), in particular the labelling moieties, associated with the target analyte in the optical readout.

The marker and the method have the same advantages as the affinity reagent. Further, the marker and the method may be supplemented with the features of the affinity reagent described in this document, in particular, the features of the dependent claims of the affinity reagent.

### Short Description of the Figures

Hereinafter, specific embodiments are described referring to the drawings, wherein:
- Figure 1: is a schematic view of a marker comprising an affinity reagent in folded and unfolded conformations, and
- Figure 2: is a schematic view of the marker with an affinity reagent comprising reaction moieties,
- Figure 3: is a schematic view of the marker with an affinity reagent comprising a linking element, and
- Figure 4: is a schematic view of steps to cross-link a nucleic acid backbone of an affinity reagent.

### Detailed Description

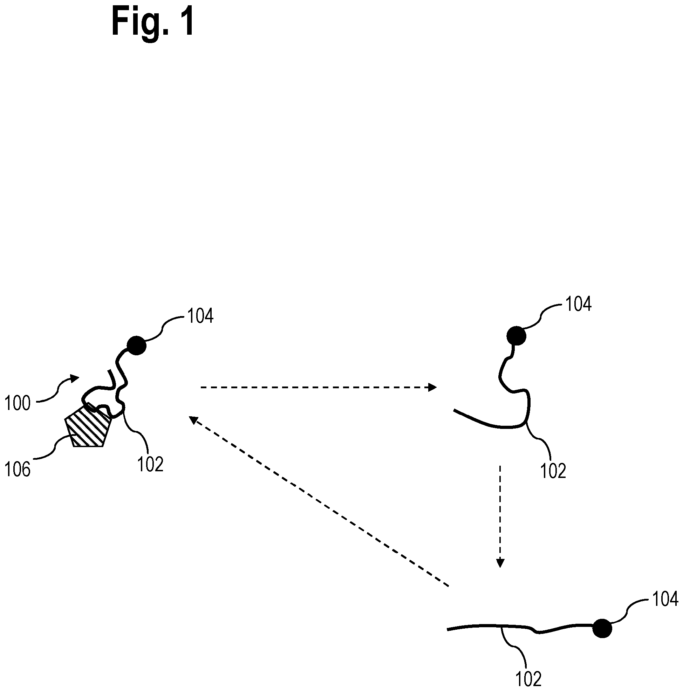

Figure 1 is a schematic view of a marker 100 comprising an affinity reagent 102 in folded and unfolded conformations. The marker 100 further comprises a label with a labelling moiety 104. When the affinity reagent 102 has a folded conformation, the affinity reagent 102, and therefore the marker 100, may specifically bind to a target analyte 106 of a biological sample. The folded conformation of the affinity reagent 102 is shown in the left view of Fig. 1. The affinity reagent 102 may be a nucleic acid based aptamer, for example.

The labelling moiety 104 may be an optically detectable moiety, such as a fluorophore. Thus, the marker 100 may be detected by means of a microscope. Alternatively, the marker 100 may comprise a plurality of labelling moieties.

Under unfolding conditions, such as at a high temperature, the affinity reagent 102 may unfold into a partially unfolded conformation shown in the top right view of Fig. 1, or into a fully unfolded conformation shown in the bottom right view of Fig. 1. Upon removal of the unfolding condition, the affinity reagent 102 may return to the folded conformation. It is only in the folded conformation of the affinity reagent 102 that the affinity reagent 102 specifically binds to the target analyte 106.

To keep the affinity reagent 102 of the marker 100 in folded conformation, in which the affinity reagent 102 specifically binds to the target analyte 106, the affinity reagent 102 may comprise at least one pair of reaction moieties. These reaction moieties may cross-link the affinity reagent 102 in order to maintain the complex structure of the affinity reagent 102. These aspects are further described below.

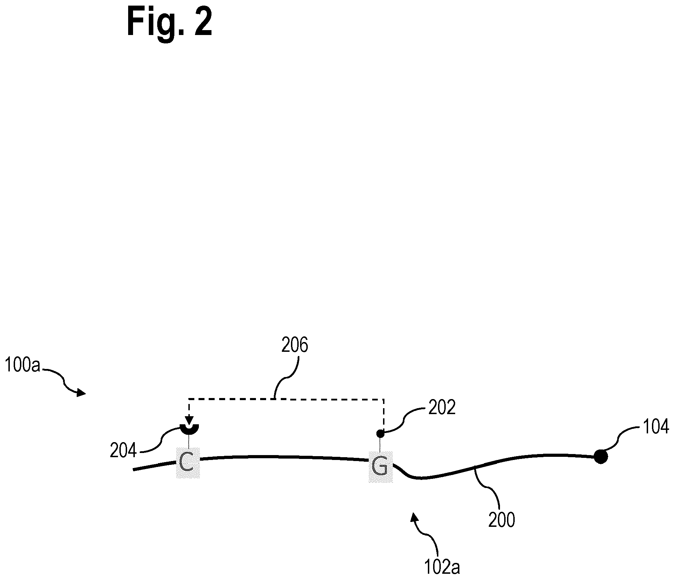

Figure 2 is a schematic view of a marker 100a with an affinity reagent 102a comprising a nucleic acid backbone 200 and reaction moieties 202, 204 of a pair of reaction moieties. The nucleic acid backbone 200 of the affinity reagent 102a is configured to specifically bind to a particular target analyte, such as the target analyte 106, for example a protein of a biological sample. In particular, the nucleic acid backbone 200 may form or fold into a complex structure or conformation. The complex structure of the backbone 200 of the affinity reagent 102a may be a secondary, tertiary and/or quaternary structure of the backbone 200. Thus, the backbone 200 of the affinity reagent 102a has a secondary, tertiary or quaternary structure, in particular. The affinity reagent 102a binds specifically to the target analyte 106 due to the complex structure of the backbone 200 of the affinity reagent 102a. Specifically, individual, discontinuous nucleotides of the nucleic acid of the backbone 200 bind to the target analyte 106. This binding may comprise intermolecular forces such as hydrogen bonding, dipole-dipole interactions, and van der Waals forces. This is in contrast to a nucleic acid hybridising, which is characterised by a continuous sequence of nucleotides of the nucleic acid hybridising to a target analyte, in particular a complementary nucleotide sequence of the target analyte.

The binding of the backbone 200 of the affinity reagent 102a to the target analyte 106 based on the complex structure of the backbone 200 enables particular high affinity binding and specificity to the target analyte 106.

The nucleic acid of the backbone 200 may comprise multiple individual sequences or strands of a nucleic acid. These individual sequences may bind to each other and form a quaternary structure.

For clarity of the Fig. 2, the nucleic acid backbone 200 is shown as an unfolded, linear structure. When the nucleic acid backbone 200 of the affinity reagent 102a of the marker 100a has the complex structure, the reaction moieties 202, 204 are in proximity to form a covalent bond. The covalent bond is indicated by reference sign 206 in Fig. 2. The covalent bond between the reaction moieties 202, 204 may cross-link the nucleic acid backbone 200 of the affinity reagent 102a to maintain the complex structure of the nucleic acid backbone 200.

The reaction moieties 202, 204 are modified nucleotides of the nucleic acid backbone 200. For example, the reaction moieties 202, 204 may comprise click chemistry groups, such as CuAAC with azide/alkyne groups or SpAAC with DBCO/azide groups, attached to nucleobases Guanine or Cytosine, respectively.

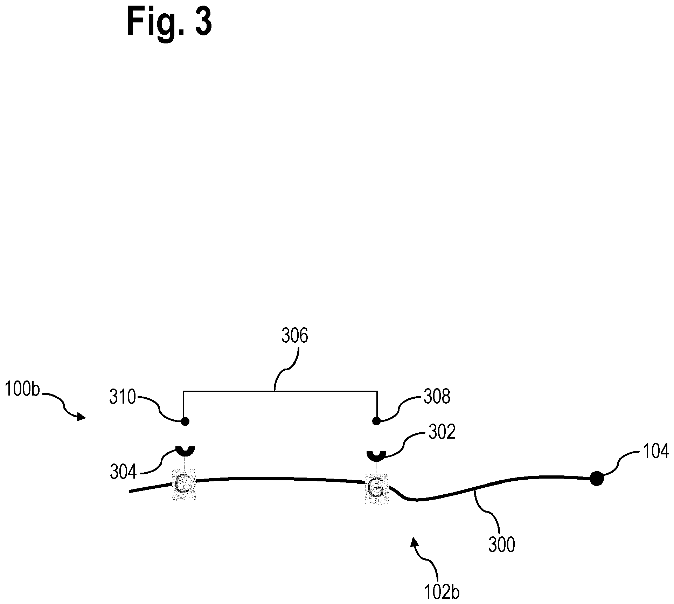

Figure 3 is a schematic view of a marker 100b with an affinity reagent 102b comprising a nucleic acid backbone 300, reaction moieties 302, 304 of a pair of reaction moieties, and a linking element 306. The nucleic acid backbone 300 of the affinity reagent 102b is configured to specifically bind to a particular target analyte, such as the target analyte 106.

As explained for the nucleic acid backbone 200 above, the nucleic acid backbone 300 may form or fold into a complex structure or conformation. For clarity of the Fig. 3, the nucleic acid backbone 300 is shown as an unfolded, linear structure. When the nucleic acid backbone 300 of the affinity reagent 102b of the marker 100b has the complex structure, the reaction moieties 302, 304 are in proximity and the linking element 306, in particular corresponding reaction moieties 308, 310 of the linking element 306, may react with the reaction moieties 302, 304 of the nucleic acid backbone 300 in order to cross-link the nucleic acid backbone 300. This enables maintaining the complex structure of the nucleic acid backbone 300. The reaction moieties 302, 304, 308, 310 may be click chemistry groups that react with each other to form covalent bonds between the reaction moieties 302 and 308 as well as between reaction moieties 304 and 310.

In particular, the linking element 306 enables linking the reaction moieties 302, 304 that are at a distance from each other when the nucleic acid backbone 300 has its complex structure that is too great for the reaction moieties 302, 304 to react with each other directly. The length of the linking element 306 may be chosen depending on the distance between the reaction moieties 302, 304 when the nucleic acid backbone 300 has its complex structure.

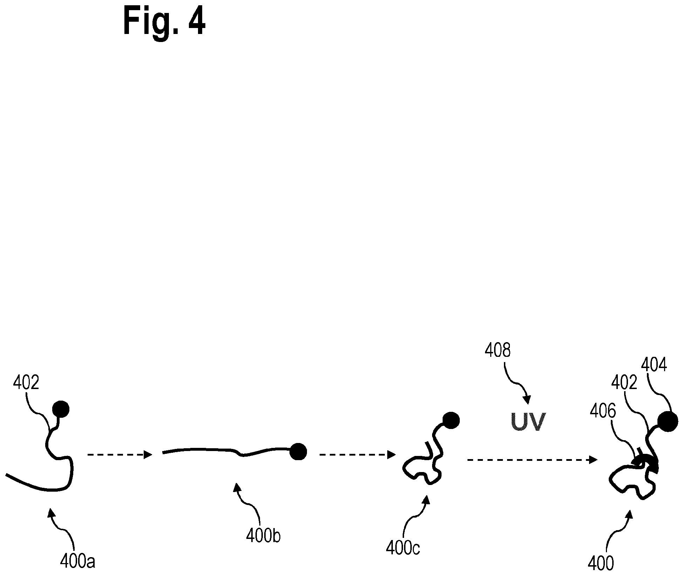

Figure 4 is a schematic view of steps to cross-link a nucleic acid backbone 402 of an affinity reagent of a marker 400 in its complex structure. The marker 400 further comprises a labelling moiety 404. The nucleic acid backbone 402 comprises a pair of reaction moieties 406 that cross-links the nucleic acid backbone 402.

Initially, a partially folded conformation 400a of the marker 400, in particular the nucleic acid backbone 402, may be fully unfolded by applying an unfolding condition such as a high temperature. This generates an unfolded, linear conformation 400b of the marker 400. The partially folded conformation 400a may alternatively be a misfolded conformation, which does not specifically bind to a particular target analyte. Subsequently, the unfolded conformation 400b may be refolded into a native complex structure 400c of the marker 400 by applying a folding condition, such as a low, or physiological temperature. The complex structure 400c may bind specifically to the particular target analyte with high affinity. However, the complex structure 400c is still prone to unfolding into the partially or fully unfolded conformations 400a, 400b. This may be caused by applying the unfolding condition or randomly due to environmental influence or storage conditions.

In order to maintain the complex structure 400c of the affinity reagent of the marker 400, the nucleic acid backbone 402 may be cross-linked. The pair of reaction moieties 406 are configured to react with each other under a catalytic condition, for example UV-light 408. After folding into the complex structure 400c, the UV-light 408 may be applied in order to react the pair of reaction moieties 406 and form a cross-link. This enables maintaining the complex structure of the affinity reagent of the marker 400 and avoids undesired unfolding of the affinity reagent of the marker 400.

In an alternative, the complex structure 400c may be refolded without the labelling moiety 404 attached to the nucleic acid backbone 402 and the pair of reaction moieties 406 may be cross-linked. Only in a subsequent step, the labelling moiety 404 may be attached to the nucleic acid backbone 402. This avoids damaging the labelling moiety 404, for example by the catalytic condition or the unfolding condition.

The marker 100, 100a, 100b, 400 may be applied in a method for analysing a biological sample. The marker 100, 100a, 100b, 400, in particular the respective affinity reagent, is specific to a target analyte of the biological sample. When introducing the marker 100, 100a, 100b, 400 into the biological sample, the marker 100, 100a, 100b, 400 binds specifically to the target analyte. Subsequently a readout, in particular an optical readout, may be generated of the biological sample with the marker 100, 100a, 100b, 400, for example, by means of a microscope. The respective labelling moiety of the marker 100, 100a, 100b, 400 may be detected in the readout and the presence or location of the target analyte in the biological sample may be determined. Preferably, the marker 100, 100a, 100b, 400 is generated prior to or during introducing the marker 100, 100a, 100b, 400 into the biological sample, in particular, as described above.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 100, 100a, 100b, 400: Marker
- 102, 102a, 102b: Affinity reagent
- 104, 404: Labelling moiety
- 106: Target analyte
- 200, 300, 402: Nucleic acid backbone
- 202, 204, 302, 304, 308, 310: Reaction moiety
- 206: Covalent bond
- 306: Linking element
- 400a: Partially unfolded conformation
- 400b: Unfolded conformation
- 400c: Complex structure
- 406: Pair of reaction moieties
- 408: Catalytic condition

## Claims

1. An affinity reagent (102, 102a, 102b) for analysing a biological sample comprising:
a nucleic acid backbone (200, 300, 402),
wherein the nucleic acid backbone (200, 300, 402) is configured to specifically bind to a target analyte (106) by its complex structure (400c), and
wherein the nucleic acid backbone (200, 300, 402) comprises at least one pair of reaction moieties (202, 204, 302, 304, 308, 310) for cross-linking the nucleic acid backbone (200, 300, 402).

2. The affinity reagent according to claim 1, wherein the reaction moieties (202, 204, 302, 304, 308, 310) of the pair of reaction moieties are configured to react with each other.

3. The affinity reagent according to one of the preceding claims, wherein the reaction moieties (202, 204, 302, 304, 308, 310) of the pair of reaction moieties are configured to react under a catalytic condition (408).

4. The affinity reagent according to one of the preceding claims further comprising a linking element (306), wherein the reaction moieties (202, 204, 302, 304, 308, 310) of the pair of reaction moieties are configured to react with corresponding reaction moieties (202, 204, 302, 304, 308, 310) of the linking element (306).

5. The affinity reagent according to one of the preceding claims, wherein the reaction moieties (202, 204, 302, 304, 308, 310) of the pair of reaction moieties are modified nucleotides.

6. The affinity reagent according to one of the preceding claims, wherein the reaction moieties (202, 204, 302, 304, 308, 310) of the pair of reaction moieties comprise click chemistry groups.

7. The affinity reagent according to one of the preceding claims, wherein the reaction moieties (202, 204, 302, 304, 308, 310) of the pair of reaction moieties are photoreactive.

8. The affinity reagent according to one of the preceding claims, wherein the nucleic acid backbone (200, 300, 402) comprises 10 to 100 nucleotides.

9. The affinity reagent according to one of the preceding claims, wherein the nucleic acid backbone (200, 300, 402) is an aptamer.

10. The affinity reagent according to one of the preceding claims, wherein the reaction moieties (202, 204, 302, 304, 308, 310) of the pair of reaction moieties are separated from each other along the nucleic acid backbone (200, 300, 402).

11. The affinity reagent according to one of the preceding claims, wherein the reaction moieties (202, 204, 302, 304, 308, 310) of the pair of reaction moieties are in proximity when the affinity reagent has its complex structure (400c).

12. The affinity reagent according to one of the preceding claims, comprising a second pair of reaction moieties, wherein the reaction moieties of the second pair differ from the reaction moieties of the at least one pair of reaction moieties.

13. A marker (100, 100a, 100b, 400) for analysing a biological sample comprising:
an affinity reagent (102, 102a, 102b) according to one of the preceding claims, and
a label comprising at least one labelling moiety (104, 404).

14. A method for analysing a biological sample comprising the steps:
introducing into the biological sample at least one marker (100, 100a, 100b, 400) according to claim 13, and
generating a readout of the biological sample with the marker (100, 100a, 100b, 400).

15. The method according to claim 14, wherein prior to introducing the at least one marker, the affinity reagent of the marker (100, 100a, 100b, 400) is generated by cross-linking a pair of reaction moieties of the marker.
